# EUROPEAN PATENT SPECIFICATION

(11) **EP 1 913 943 B1**
(45) Date of publication and mention of the grant of the patent: **21.09.2011**
(21) Application number: 06767384.8
(22) Date of filing: 27.06.2006
(51) Int. Cl.: A61K 31/198, A23K 1/16, A23L 1/305, A61K 31/7016, A61P 13/02

(54) **PROPHYLACTIC OR THERAPEUTIC COMPOSITION FOR HEMOGLOBINURIA OR MYOGLOBINURIA**
PROPHYLAKTISCHE ODER THERAPEUTISCHE ZUSAMMENSETZUNG GEGEN HÄMOGLOBINURIE ODER MYOGLOBINURIE
COMPOSITION PROPHYLACTIQUE OU THERAPEUTIQUE POUR LUTTER CONTRE L'HEMOGLOBINURIE OU LA MYOGLOBINURIE

(30) Priority: 27.06.2005 JP 2005186114
(43) Date of publication of application: 23.04.2008
(73) Proprietor: Kyowa Hakko Bio Co., Ltd., Tokyo, 100-8185 (JP)
(72) Inventor: SHIMADA, Kenjiro, Ibaraki 300-0836 (JP); TANIOKA, Tsuyoshi, Hidaka-gun Hokkaido 056-0003 (JP)
(74) Representative: Dossmann, Gérard
(86) International application number: PCT/JP2006/312767
(87) International publication number: WO 2007/000985

(56) References cited:
- WO-A-2004/049830
- JP-A- 06 070 718
- JP-A- 08 198 748
- JP-A- 2000 072 669
- CHANDER V. ET AL.: 'Molsidomine, a nitric oxide donor and L-arginine protects against rhabdomyolysis-induced myoglobinuric acute renal failure' BIOCHIMICA ET BIOPHYSICA ACTA, GENERAL SUBJECTS vol. 1723, no. 1-3, 2005, pages 208 - 214, XP004910601

## Description

### Technical Field

The present invention relates to a prophylactic or therapeutic composition for hemoglobinuria or myoglobinuria, which comprises a branched-chain amino acid or a salt thereof, the basic amino acid ornithine or a salt thereof, glutamine or a salt thereof and trehalose as active ingredients.

### Background Art

In humans or animals, after they exercise hard or when they are in an extremely fatigued state, etc., hemolysis occurs in the blood, which leads to hemoglobinuria, further, skeletal muscle is broken down, which leads to myoglobinuria. In such a circumstance, however, there is no means for preventing hemoglobinuria or myoglobinuria except that one should be careful not to apply a load of exercise or the like. Further, in the treatment of hemoglobinuria or myoglobinuria, a steroid has been used, however, it has problems such as side effects.

Valine, leucine and isoleucine are called a branched-chain amino acid, and a composition which contains such three types of amino acids is known to have an effect of relieving muscle fatigue (see Patent document 1). Further, a basic amino acid such as ornithine or arginine is known to have an effect of building muscle (see Non-patent document 1). Further, glutamine is known to be effective in skeletal muscle atrophy (see Patent document 2). Further, a composition which comprises valine, leucine, isoleucine, glutamine and a whey protein component is known to have an effect of improving long-lasting muscle fatigue (see Patent document 3).
[Patent document 1] Japanese Published Unexamined Patent Application No. 198748/1996
[Patent document 2] Japanese Published Examined Patent Application No. 94389/1995
[Patent document 3] Japanese Published Unexamined Patent Application No. 182630/2004
[Non-patent document 1] "The Journal of Sports Medicine and Physical Fitness", Vol. 29, No. 1, pp. 52-56, 1989 JP 2000-072669 discloses the treatment of the reductivate of the blood levels of aminoacids associated withe severe exercise with a composition comprising trehalose and branched aminoacids

### Disclosure of the invention

### Problems that the Invention is to Solve

An object of the present invention is to provide a prophylactic or therapeutic composition for hemoglobinuria or myoglobinuria which may occur in humans or animals upon loading of exercise or stress or the like.

### Means for Solving the Problems

The present invention relates to the following (1) to (7).
(1) A prophylactic or therapeutic composition for hemoglobinuria or myoglobinuria, which comprises a branched-chain amino acid or a salt thereof, the basic amino acid ornithine or a salt thereof, glutamine or a salt thereof and trehalose as active ingredients.
(2) The composition according to the above (1), wherein the branched-chain amino acid is valine, leucine or isoleucine.

(3) The composition according to any one of the above (1) to (2), wherein the composition is a pharmaceutical composition, a food or drink, a food additive, a feed or a feed additive.
(4) A method for preventing or treating hemoglobinuria or myoglobinuria, which comprises administering to a subject in need thereof, or allowing the subject to ingest, an effective amount of a branched-chain amino acid or a salt thereof, the basic amino acid ornithine or a salt thereof, glutamine or a salt thereof and trehalose
(5) The method according to the above (4) wherein the branched-chain amino acid is valine, leucine or isoleucine.

(6) Use of a branched-chain amino acid or a salt thereof, the basic amino acid ornithine or a salt thereof, glutamine or a salt thereof and trehalose for the manufacture of a prophylactic or therapeutic composition for hemoglobinuria or myoglobinuria
(7) The use according to the above (6), wherein the branched-chain amino acid is valine, leucine or isoleucine.

### Effect of the invention

According to the present invention, a safe and effective prophylactic or therapeutic composition for hemoglobinuria or myoglobinuria, which comprises a branched-chain amino acid or a salt thereof, the basic amino acid ornithine or a salt thereof, glutamine or a salt thereof, and trehalose as active ingredients can be provided.

### Best Mode for Carrying Out the Invention

As the branched-chain amino acid to be used in the present invention, preferred is a single substance of valine, leucine or isoleucine, or a mixture of two to three types of valine, leucine and isoleucine. In a mixture of three types thereof, the weight ratio of the respective components is preferably 0.5 to 1.5:1 to 3:0.5 to 1.5.
The basic amino acid to be used in the present invention is ornithine,

The weight ratio of the branched-chain amino acid or a salt thereof, the basic amino acid ornithine or a salt thereof, and glutamine or a salt thereof in the composition of the present invention is preferably 1:0.5 to 5:0.5 to 5, more preferably 1:0.8. to 2.0:0.8 to 2.0, particularly preferably 1:0.9 to 1.5:0.9 to 1.5.
With regard to the branched-chain amino acid, the basic amino acid and glutamine, any of L-form, D-form and a mixture of L-form and D-form may be used in each case, however, L-form is preferably used.

Examples of the salts of branched-chain amino acid, the basic amino acid and glutamine include acid addition salts, metal salts, ammonium salts, organic amine addition salts, amino acid addition salts and the like.
Examples of the acid addition salts include inorganic acid salts such as hydrochlorides, sulfates, nitrates and phosphates, and organic acid salts such as acetates, maleates, fumarates, citrates, malates, lactates, α-ketoglutarates, gluconates and caprylates.

Examples of the metal salts include alkali metal salts such as sodium salts and potassium salts, alkaline earth metal salts such as magnesium salts and calcium salts, aluminum salts, zinc salts and the like.
Examples of the ammonium salts include salts of ammonium, tetramethylanmonium and the like.
Examples of the organic amine addition salts include salts of morpholine, piperidine and the like.

Examples of the amino acid addition salts include salts of glycine, phenylalanine, lysine, aspartic acid, glutamic acid and the like.
In the composition of the present invention, the branched-chain amino acid or a salt thereof, the basic amino acid ornithine or a salt thereof and glutamine or a salt thereof described above are incorporated as active ingredients, and further trehalose is incorporated. Trehalose is incorporated in an amount of preferably 0.1 to 20% by weight, more preferably 0.5 to 15% by weight, particularly preferably 1 to 10% by weight based on the total amount of the branched-chain amino acid or a salt thereof, the basic amino acid ornithine or a salt thereof and glutamine or a salt thereof.

Further, the composition of the present invention may be prepared in such a manner that the branched-chain amino acid or a salt thereof, the basic amino acid ornithine or a salt thereof, glutamine or a salt thereof and trehalose are contained in the same composition. However, separate compositions each of which contains one substance or two or more substances selected from the branehed-chain amino acid or a salt thereof, the basic amino acid ornithine or a salt thereof glutamine or a salt thereof and trehalose, are produced and can be used as compositions in a form of a kit or a set (hereinafter also referred to simply as a kit or the like).

Examples of the combination of compositions constituting the kit or the like include a combination of a composition containing the branched-chain amino acid or a salt thereof, a composition containing the basic amino acid ornithine or a salt thereof, and a composition containing glutamine or a salt thereof; a combination of a composition containing the branched-chain amino acid or a salt thereof and the basic amino acid ornithine or a salt thereof and a composition containing glutamine or a salt thereof; a combination of a composition containing the branched-chain amino acid or a salt thereof and glutamine or a salt thereof and a composition containing the basic amino acid ornithine or a salt thereof; a combination of a composition containing the branched-chain amino acid or a salt thereof and a composition containing the basic amino acid ornithine or a salt thereof and glutamine or a salt thereof;

Each composition contained in the kit or the like may be present in any state as long as it is a state where each composition is present separately. For example, each composition may be separately packed or may be mixed in the same container.
When each composition contained in the kit or the like is separately administered or ingested, it is desirable that administration is conducted within a period where the active ingredient in the composition has a high efficacy *in vivo.* For example, all compositions are administered or ingested within 8 hours, preferably within 2 hours for one administration or ingestion.

The composition of the present invention can be used as a pharmaceutical composition, a food or drink, a food additive, a feed or a feed additive (hereinafter, also referred to as the pharmaceutical composition, food or drink, food additive, feed or feed additive of the present invention).
In the case where the composition of the present invention is used as a pharmaceutical composition, the branched-chain amino acid or a salt thereof, the basic amino acid ornithine or a salt thereof, glutamine or a salt thereof and trehalose can be administered as such, however, usually it is desirable that they are provided as any of various kinds of preparations.

The preparation contains the branched-chain amino acid or a salt thereof, the basic amino acid ornithine or a salt thereof glutamine or a salt thereof and trehalose as active ingredients, however, it may contain any other active ingredients for the therapy. Further, these pharmaceutical preparations may be produced by any method well known in the technical field of pharmaceutics by mixing the active ingredients with one or more pharmaceutically acceptable carriers.

With regard to the route of administration of the preparation, it is desirable to select a route of administration that is the most effective for the therapy, and examples thereof include oral administration and parenteral administration such as intravenous administration, intraperitoneal administration or subcutaneous administration, however, oral administration is preferred.
With regard to the dosage form, any of oral preparations such as tablets, powders, granules, pills, suspensions, emulsions, infusions, decoctions, capsules, syrups, liquid preparations, elixirs, extracts, tinctures and fluid extracts and parenteral preparations such as injections, drippings, creams and suppositories may be used, however, oral preparations are preferably used.

For example, a liquid preparation such as a syrup, which is suitable for oral administration, can be prepared by adding water, a saccharide such as sucrose, sorbitol or fructose, a glycol such as polyethylene glycol or propylene glycol, an oil such as sesame oil, olive oil or soybean oil, an antiseptic such as a p-hydroxybenzoate ester, a preservative such as a p-oxybenzoate derivative (e.g., methyl paraoxybenzoate) or sodium benzoate, a flavor such as strawberry flavor or peppermint, or the like.

Further, for example, tablets, powders or granules, each of which is suitable for oral administration, can be prepared by adding a saccharide such as lactose, sugar, glucose, sucrose, mannitol or sorbitol, starch such as that of potato, wheat or corn, an inorganic substance such as calcium carbonate, calcium sulfate, sodium hydrogen carbonate or sodium chloride, an excipient such as crystalline cellulose or plant powder (e.g., licorice root powder, gentian powder or the like), a disintegrator such as starch, agar, gelatin powder, crystalline cellulose, carmellose sodium, carmellose calcium, calcium carbonate, sodium hydrogen carbonate or sodium alginate, a lubricant such as magnesium stearate, talc, hydrogenated plant oil, Macrogol or silicone oil, a binder such as polyvinyl alcohol, hydroxypropyl cellulose, methyl cellulose, ethyl cellulose, carmellose, gelatin or starch paste, a surfactant such as a fatty acid ester, a plasticizer such as glycerol, or the like.

For example, an injection, which is suitable for parenteral administration, preferably comprises a sterilized aqueous preparation containing the branched-chain amino acid or a salt thereof, the basic amino acid or a salt thereof and glutamine or a salt thereof, which is isotonic to the recipient's blood. In the case of an injection, for example, a solution for injection is prepared using a carrier comprising a salt solution, a glucose solution, or a mixture of a salt solution and a glucose solution, or the like.
Further, also in these parenteral preparations, one or more auxiliary components selected from the antiseptics, preservatives, flavors, excipients, disintegrators, lubricants, binders, surfactants and plasticizers described in the examples of the oral preparations, and the like.

The concentration of the branched-chain amino acid or a salt thereof, the basic amino acid ornithine or a salt thereof glutamine or a salt thereof and trehalose in the pharmaceutical composition of the present invention is appropriately selected depending on the type of preparation, the effect expected by administration of the preparation, and the like, however, the concentration of the branched-chain amino acid or a salt thereof, the basic amino acid ornithine or a salt thereof and glutamine or a salt thereof and trehalose is usually 0.1 to 100% by weight, preferably, 0.5 to 70% by weight, particularly preferably 1 to 50% by weight.
The dose and the administration frequency of the pharmaceutical composition of the present invention may vary depending on the administration form, the age and the body weight of the patient, and the nature or the severity of the symptom to be treated. In general, it is administered once to several times a day in an amount of usually 50 mg to 30 g, preferably 100 mg to 10 g, more preferably 200 mg to 3 g per day for an adult in terms of the branched-chain amino acid or a salt thereof, the basic amino acid ornithine or a salt thereof, glutamine or a salt thereof and trehalose.

The administration period is not particularly limited, however, it is usually one day to one year, preferably, one week to three months.
The pharmaceutical composition of the present invention can be used not only for humans, but also for animals other than humans (hereinafter, simply referred to as non-human animals).
Examples of the non-human animals include animals other than humans such as mammals, birds, reptiles, amphibians and fishes.

In the case of administering the pharmaceutical composition to non-human animals, the dose thereof may vary depending on the age and the kind of the animal, and the nature or the severity of the symptom. In general, it is administered once to several times a day in an amount of usually 1 to 600 mg, preferably 2 to 200 mg, more preferably 4 to 60 mg per day per 1 kg of the body weight in terms of the branched-chain amino acid or a salt thereof, the basic amino acid ornithine or a salt thereof glutamine or a salt thereof, and trehalose.

The administration period is not particularly limited, however, it is usually one day to one year, preferably, one week to three months.
The food additive containing the branched-chain amino acid or a salt thereof , the basic amino acid ornithine or a salt thereof glutamine or a salt thereof and trehalose as active ingredients can be prepared in the same manner as the above-mentioned pharmaceutical composition.

The food additive of the present invention can be processed and produced in a form of powder, granules, pellets, tablets and various kinds of liquid preparations by, if necessary, mixing or dissolving other food additives therein.
The food or drink of the present invention can be processed and produced by a common production process for foods or drinks except that the branched-chain amino acid or a salt thereof the basic amino acid ornithine or a salt thereof, glutamine or a salt thereof and trehalose, or the food additive of the present invention are added to a food or drink.

The food or drink of the present invention can also be produced by a granulation method such as fluid bed granulation, stirring granulation, extrusion granulation, oscillating granulation, gas stream granulation, compression molding granulation, disruption granulation, spray granulation or jet granulation; a coating method such as pan coating, fluid bed coating or dry coating; a swelling method such as puff drying, an excess steam method, a foam mat method or a microwave heating method; an extrusion method such as using an extruding granulator or an extruder; or the like.

The food or drink of the present invention may be in any forms such as juice, soft drinks, tea, lactic acid bacteria beverage, milk products such as fermented milk, ice cream, butter, cheese, yogurt, processed milk and skim milk, meat products such as ham, sausage and hamburger, fish paste foods such as kamaboko (boiled fish paste), chikuwa (a kind of Japanese fish sausage) and satsumaage (deep-fried fish ball containing vegetable bits), egg products such as dashimaki (omelet with stock) and tamago-dofu (steamed beaten egg with soup stock), confectionary such as cookies, jelly, chewing gum, candy and snacks, bread, noodles, pickles, smoked fish and meat, dried fish, tsukudani (simmered meat in soy sauce and sugar), salted products, soup, seasonings, and the like.

The food or drink of the present invention may also be in a form such as a powdered food, a sheet-shaped food, a bottled food, a canned food, a retort food, a capsule food, a tablet food, a liquid food or a drinkable preparation.
The food or drink of the present invention can be used as a food or drink such as a health food, a functional food, a nutritional supplement or a food for specified health use for preventing or treating hemoglobinuria or myoglobinuria.

To the food or drink or the food additive of the present invention, an additive generally used in foods or drinks, for example, a sweetener, a coloring agent, a preservative, a thickening stabilizer, an antioxidant, a color-developing agent, a bleaching agent, an anti-fungal agent, a gum base, a bitter agent, an enzyme, a wax, a sour agent, a seasoning, an emulsifier, a nutrient supplement, an additional material for preparation, a flavor, a spice extract or the like described in Japan's Specifications And Standards For Food Additives (Japan Food Additives Association, issued on January 6, 1997) may be added.

The amount of the branched-chain amino acid or a salt thereof, the basic amino acid ornithine or a salt thereof glutamine or a salt thereof and trehalose or the food additive to be added to the food or drink of the present invention is appropriately selected depending on the type of food or drink, the effect expected by ingestion of the food or drink, and the like, however, the branched-chain amino acid or a salt thereof, the basic amino acid ornithine or a salt thereof glutamine or a salt thereof and trehalose are usually added thereto in an amount of 0.1 to 100% by weight, preferably, 0.5 to 70% by weight, particularly preferably 1 to 50% by weight.

The ingestion amount of the food or drink of the present invention may vary depending on the ingestion form, the age and the body weight of the ingesting person, and the like. In general, it is ingested once to several times a day in an amount of usually 50 mg to 30 g, preferably 100 mg to 10 g, more preferably 200 mg to 3 g per day for an adult in terms of the branched-chain amino acid or a salt thereof, the basic amino acid ornithine or a salt thereof, glutamine or a salt thereof and trehalose.
The ingestion period is not particularly limited, however, it is usually one day to one year, preferably, one week to three months.

The feed additive containing the branched-chain amino acid or a salt thereof , the basic amino acid ornithine or a salt thereof glutamine or a salt thereof, and trehalose as active ingredients can be prepared in the same manner as the food additive of the present invention. The feed additive of the present invention can be processed and produced in a form of powder, granules, pellets, tablets and various kinds of liquid preparations by, if necessary, mixing or dissolving other feed additives therein.
The feed of the present invention can be processed and produced by a common production process for feeds except that the branched-chain amino acid or a salt thereof, the basic amino acid ornithine or a salt thereof, glutamine or a salt thereof and trehalose or the feed additive of the present invention are added to a feed for non-human animals.

The feed for non-human animals may be a feed for non-human animals such as mammals, birds, reptiles, amphibians and fishes. Examples thereof include feeds for pets such as dogs, cats and mice, feeds for livestock such as cattle and pigs, feeds for poultry such as domestic fowls and turkeys, feeds for cultivated fishes such as sea breams and young yellowtails, and the like.
Examples of the feed to which the branched-chain amino acid or a salt thereof, the basic amino acid ornithine or a salt thereof glutamine or a salt thereof and trehalose or the feed additive of the present invention are added include cereals, chaff and bran, vegetable oil cakes, animal feed materials, other feed materials, purified products, and the like.

Examples of the cereals include milo, wheat, barley, oats, rye, brown rice, buckwheat, foxtail millet, broomoorn millet, Japanese millet, corn, soybean, and the like.
Examples of the chaff and bran include rice bran, defatted rice bran, wheat bran, wheat middlings, wheat germ, barely bran, pellets, corn bran, corn germ, and the like.
Examples of the vegetable oil cakes include soybean oil cake, soybean flour, linseed oil cake, cotton seed oil cake peanut oil cake, safflower oil cake, coconut oil cake, palm oil cake, sesame oil cake, sunflower oil cake, rapeseed oil cake, kapok oil cake, mustard seed oil cake, and the like.

Examples of the animal feed materials include fish meal (such as northern ocean meal, imported meal, whole meal and coastal meal), fish soluble, meat meal, meat and bone meal, blood powder, degraded hair, bone meal, processed by-products for livestock, feather meal, silk-worm pupa, skim milk powder, casein, dry whey, and the like.
Examples of the other feed materials include stems and leaves of plants (such as alfalfa, hay cube, alfalfa leaf meal and the powder of false acacia), processed industrial by-products of corn (such as corn gluten meal, corn gluten feed and corn steep liquor), processed starch products (such as starch), sugar, fermentation industrial products (such as yeast, beer cake, malt root, alcohol cake and soy source cake), agricultural by-products (such as processed citrus fruit cake, soybean curd cake, coffee cake and cocoa cake), cassava, broad bean, guar meal, sea weed, krill, spirulina, chlorella, minerals, and the like.

Examples of the purified products include proteins (such as casein and albumin), amino acids, sugars (such as starch, cellulose, sucrose and glucose), minerals, vitamins, and the like.
The feed of the present invention may also be produced by a granulation method such as fluid bed granulation, stirring granulation, extrusion granulation, oscillating granulation, gas stream granulation, compression molding granulation, disruption granulation, spray granulation, or jet granulation; a coating method such as pan coating, fluid bed coating or dry coating; a swelling method such as puff drying, an excess steam method, a foam mat method or a microwave heating method; an extrusion method such as using an extruding granulator or an extruder; or the like.

The feed of the present invention can be used as a feed for preventing or treating hemoglobinuria or myoglobinuria.
The amount of the branched-chain amino acid or a salt thereof, the basic amino acid ornithine or a salt thereof, glutamine or a salt thereof and trehalose, or the feed additive of the present invention to be added to the feed of the present invention is appropriately selected depending on the type of feed, the effect expected by ingestion of the feed, and the like, however the branched-chain amino acid or a salt thereof, the basic amino acid ornithine or a salt thereof, glutamine or a salt thereof and trehalose are usually added thereto in an amount of 0.1 to 100% by weight, preferably, 0.5 to 70% by weight, particularly preferably 1 to 50% by weight.

In the case where a non-human animal is allowed to ingest the feed of the present invention, the ingestion amount thereof may vary depending on the ingestion form, the type of the ingesting animal, the age and the body weight of the animal, and the like. In general, the ingestion amount thereof is 1 to 600 mg, preferably 2 to 200 mg, more preferably 4 to 60 mg per day per 1 kg of the body weight in terms of the branched-chain amino acid or a salt thereof, the basic amino acid ornithine or a salt thereof, glutamine or a salt thereof, and trehalose.
The ingestion period is not particularly limited, however, it is usually one day to one year, preferably, one week to three months.

By administering the composition of the present invention to humans or non-human animals, or by allowing humans or non-human animals to ingest the composition of the present invention according to the above-mentioned method, hemoglobinuria or myoglobinuria in the humans or non-human animals can be prevented or treated.
Hereinafter, test examples in which prophylactic and therapeutic effects of the composition of the present invention on hemoglobinuria and myoglobinuria were examined are shown.

### Test example 1

To a racehorse (male, two years old with a body weight of 480 kg) which developed severe hemoglobinuria and myoglobinuria and had difficulty in running, the composition of Example 1 was orally administered at a dose of 50 g on day 1 and at a dose of 30 g per day on days 2 to 4, and walking conditions and urine were examined. As a result, on day 4 and thereafter, the symptoms of hemoglobinuria and myoglobinuria disappeared and the horse was able to run again.

### Test example 2

To 11 racehorses, the composition of Example 1 was orally administered at a dose of 10 to 15 g per day continuously. As a result, although the racehorses had harsh "oikiri" (which means a training carried out several days before a race, and in which the running time is measured and the conditions of the racehorses are checked), the onset of hemoglobinuria and myoglobinuria was not observed at all.
Hereinafter, Examples of the present invention are shown.

### Example 1

To an amino acid mixture comprising 250 g of leucine, 125 g of isoleucine, 125 g of valine, 500 g of glutamine and 500 g of ornithine, all of which had been granulated, 75 g of trehalose was added and mixed therein, whereby a nutritional supplement was produced.

### Example 2

To an amino acid mixture comprising 250 g of leucine, 125 g of isoleucine, 125 g of valine, 500 g of glutamine and 500 g of ornithine, all of which had been granulated, 950 g of erythritol, 50 g of sucrose fatty acid ester, 350 g of citric acid and 150 g of trehalose were added and mixed therein.
Then, by using a rotary tabletting machine with a flat pestle having a diameter of 13 mm (trade name: AP-15 Model, manufactured by Hata Iron Works Co., Ltd.), whereby tablets containing 500 mg of the above-mentioned amino acid mixture per tablet were produced.

### Industrial Applicability

According to the present invention, a safe and effective prophylactic or therapeutic composition for hemoglobinuria or myoglobinuria, which comprises a branched-chain amino acid or a salt thereof, the basic amino acid ornithine or a salt thereof glutamine or a salt thereof and trehalose as active ingredients can be provided.

## Claims

1. Composition comprising a branched-chain amino acid or a salt thereof the basic aminoacid, ornithine or a salt thereof, glutamine or a salt thereof and trehalose as active ingredients for use in the prophylaxis or the therapeutic treatment of hemoglobinuria or myoglobinuria.

2. The compositon for use according to claim 1, wherein the branched-chain amino acid is valine, leucine or isoleucine.

3. The composition for use according to any one of claims 1 to 2, wherein the composition is a pharmaceutical composition, a food or drink, a food additive, a feed or a feed additive.

4. The composition for use according to any one of claims 1 to 3 which comprises administering to a subject in need thereof, or allowing the subject to ingest, an effective amount of a branched-chain amino acid or a salt thereof, ornithine or a salt thereof, glutamine or a salt thereof and trehalose.

5. Use of a composition comprising branchod-chain amino acid or a salt thereof, the basic aminoacid ornithine or a salt thereof, glutamine or a salt thereof and trehalose for the manufacture of a medicament for the prophylaxis or the therapeutic treatment of hemoglobinuria or myoglobinuria.

6. Use according to claim 5, wherein the branched-chain amino acid is valine, leucine or isoleucine.

7. Composition which comprises a branched-chain amino acid or a salt thereof, the basic aminoacid ornithine or a salt thereof, glutamine or a salt thereof and trehalose as active ingredients contained in the same preparation or in separate preparations for is administration, optionally separately, to a subject in need thereof as a agent for the prophylaxis or the therapeutic treatment of hemoglobinuria or myoglobinuria.

## Patentansprüche

1. Zusammensetzung, die eine verzweigtkettige Aminosäure oder ein Salz davon, die basische Aminosäure Ornithin oder ein Salz davon, Glutamin oder ein Salz davon sowie Trehalose als Wirkstoffe zur Verwendung bei der Prophylaxe oder therapeutischen Behandlung von Hämoglobinurie oder Myoglobinurie umfasst.

2. Zusammensetzung zur Verwendung nach Anspruch 1, wobei die verzweigtkettige Aminosäure Valin, Leucin oder Isoleucin ist.

3. Zusammensetzung zur Verwendung nach einem der Ansprüche 1 bis 2, wobei die Zusammensetzung eine pharmazeutische Zusammensetzung, ein Lebensmittel oder Getränk, ein Lebensmittelzusatz, ein Futter oder ein Futterzusatz ist.

4. Zusammensetzung zur Verwendung nach einem der Ansprüche 1 bis 3, bei der eine wirksame Menge einer verzweigtkettigen Aminosäure oder eines Salzes davon, von Ornithin oder einem Salz davon, von Glutamin oder einem Salz davon und von Trehalose einem Patienten, der dieses braucht, verabreicht oder zugeführt wird.

5. Verwendung einer Zusammensetzung, die eine verzweigtkettige Aminosäure oder ein Salz davon, die basische Aminosäure Ornithin oder ein Salz davon, Glutamin oder ein Salz davon sowie Trehalose umfasst, für die Herstellung eines Medikaments zur Prophylaxe oder therapeutischen Behandlung von Hämoglobinurie oder Myoglobinurie.

6. Verwendung nach Anspruch 5, wobei die verzweigtkettige Aminosäure Valin, Leucin oder Isoleucin ist.

7. Zusammensetzung, die eine verzweigtkettige Aminosäure oder ein Salz davon, die basische Aminosäure Ornithin oder ein Salz davon sowie Trehalose als Wirkstoffe umfasst, die in demselben Präparat oder in getrennten Präparaten zu ihrer Verabreichung, optional getrennt voneinander, an einen Patienten, der sie braucht, enthalten sind, als Mittel zur Prophylaxe oder therapeutischen Behandlung von Hämoglobinurie oder Myoglobinurie.

## Revendications

1. Composition comprenant, comme ingrédients actifs, un acide aminé à chaine ramifiée ou un sel de celui-ci, l'acide aminé basique ornithine ou un sel de celle-ci, la glutamine ou un sel de celle-ci, ainsi que du tréhalose, en vue de son utilisation pour la prophylaxie ou le traitement thérapeutique de l'hémoglobinurie ou de la myoglobinurie.

2. Composition en vue de son utilisation selon la revendication 1, l'acide acide aminé à chaine ramifiée étant la valine, la leucine ou l'isoleucine.

3. Composition en vue de son utilisation selon l'une quelconque des revendications 1 à 2, la composition étant une composition pharmaceutique, un aliment ou une boisson, un complément alimentaire, un aliment pour animaux ou un complément alimentaire pour animaux.

4. Composition en vue de son utilisation selon l'une quelconque des revendications 1 à 3, qui comprend d'administrer à un sujet qui en a besoin ou de permettre au sujet d'ingérer une quantité efficace d'un acide aminé à chaine ramifiée ou d'un sel de celui-ci, d'omithine ou d'un sel de celle-ci, de glutamine ou d'un sel de celle-ci, ainsi que de tréhalose.

5. Utilisation d'une composition comprenant un acide aminé à chaine ramifiée ou un sel de celui-ci, l'acide aminé basique ornithine ou un sel de celle-ci, la glutamine ou un sel de celle-ci, ainsi que du tréhalose, pour la fabrication d'un médicament pour la prophylaxie ou le traitement thérapeutique de l'hémoglobinurie ou de la myoglobinurie.

6. Utilisation selon la revendication 5, l'acide acide aminé à chaine ramifiée étant la valine, la leucine ou l'isoleucine.

7. Composition comprenant, comme ingrédients actifs, un acide aminé à chaine ramifiée ou un sel de celui-ci, l'acide aminé basique ornithine ou un sel de celle-ci, la glutamine ou un sel de celle-ci, ainsi que du tréhalose, contenus dans une même préparation ou dans des préparations distinctes, en vue de leur administration, optionnellement de manière distincte, à un sujet qui en a besoin, en tant qu'agent pour la prophylaxie ou le traitement thérapeutique de l'hémoglobinurie ou de la myoglobinurie.
